# EUROPÄISCHE PATENTSCHRIFT

(11) **EP 3 092 980 B1**
(45) Veröffentlichungstag und Bekanntmachung des Hinweises auf die Patenterteilung: **15.09.2021**
(21) Anmeldenummer: 16169456.7
(22) Anmeldetag: 12.05.2016
(51) Int. Cl.: A61F 5/01, F16F 9/53, F16F 9/20

(54) **VORRICHTUNG ZUR STABILISIERUNG VON KÖRPERGELENKEN, MUSKELN UND SEHNEN**
DEVICE FOR THE STABILISATION OF BODY JOINTS, MUSCLES AND TENDON
PROCEDE DE STABILISATION D'ARTICULATIONS, DE MUSCLES ET DE VEINES

(30) Priorität: 12.05.2015 DE 102015107408
(43) Veröffentlichungstag der Anmeldung: 16.11.2016
(73) Patentinhaber: Betterguards Technology GmbH, 10625 Berlin (DE)
(72) Erfinder: BICHLER, Vinzenz, 10777 Berlin (DE); STUMPER, Timo, 12101 Berlin (DE)
(74) Vertreter: Molnia, David

(56) Entgegenhaltungen:
- WO-A1-94/12066
- WO-A1-2013/174989
- US-A- 4 471 538
- US-A- 5 450 931
- US-A- 5 712 011
- None

## Beschreibung

### Technisches Gebiet

Die vorliegende Erfindung betrifft eine Vorrichtung zur Stabilisierung von Körpergelenken, Muskeln und Sehnen umfassend einen Aufnahmekörper, wobei der Aufnahmekörper mit einem dilatanten Fluid gefüllt ist und der Aufnahmekörper sich in länglicher Form von einem ersten Ende hin zu einem zweiten Ende erstreckt, und einen Zugkörper zum Einleiten einer äußeren Kraft in die Vorrichtung.

### Stand der Technik

Es ist bekannt Körpergelenke, Muskeln und Sehnen mittels Vorrichtungen, welche eine adaptive Bewegungsbegrenzung ermöglichen zu stabilisieren. Unter anderem wird das adaptive Verhalten solcher Vorrichtungen dadurch erreicht, dass sich zwei Körper relativ zueinander bewegen, wobei sich zwischen den Körpern ein dilatantes Fluid befindet. Die sich gegenüberliegenden Flächen der Körper bilden dabei Scherflächen, welche auf Grund der Relativbewegung Scherkräfte in das dilatante Fluid einleiten. Umso größer die Scherkräfte sind umso zähflüssiger verhält sich das dilatante Fluid. Ab einer definierten Scherkraft erfährt das dilatante Fluid einen Schersprung, durch welchen der Grad der Verfestigung rapide zunimmt.

Die Vorrichtungen werden zwischen zwei Körperstellen eines Anwenders fixiert. Dabei bildet ein Scherkörper der Vorrichtung eine Aufnahme, welche mit dem dilatanten Fluid gefüllt ist. Der andere Scherkörper bildet einen Auszugskörper, welcher in der Aufnahme bewegbar angeordnet ist. Werden über die beiden Körperstellen des Anwenders physiologische Kräfte, das heißt für das entsprechend zu stabilisierende Körperteil unkritische Kräfte, in die Vorrichtung eingeleitet, so wird auf Grund des flüssigen Zustands des dilatanten Fluids eine Relativbewegung der Aufnahme und des Auszugskörper und damit eine Bewegung des zu stabilisierenden Körperteils zugelassen. Werden hingegen unphysiologische Kräfte, das heißt für das entsprechend zu stabilisierende Körperteil kritische Kräfte, in die Vorrichtung eingeleitet, rufen die von den Scherflächen der Aufnahme und des Auszugskörpers ausgehenden Scherkräfte eine Scherverfestigung des dilatanten Fliuds hervor, durch welche keine Relativbewegung zwischen dem Auszugskörper und der Aufnahme mehr möglich ist.

Eine solche Vorrichtung ist beispielsweise aus der WO 2013/174989 A1 bekannt, welche eine orthopädische Vorrichtung zur Begrenzung der Bewegung eines zwischen einem ersten und einem zweiten Körperbereich angeordneten Gelenks zeigt.

Da das Wirkprinzip solcher Vorrichtungen auf der Scherung zwischen der Aufnahme und dem Auszugskörper basiert, ist die Größe der Scherflächen entscheidend. Je größer die für gewöhnlich an dem zu stabilisierenden Körperteil auftretenden Kräfte sind, umso größer müssen auch die Scherflächen der Vorrichtung ausgelegt werden.

Große sich gegenüberliegende Flächen der Aufnahme und des Auszugskörpers erfordern jedoch einen größeren Bauraum. Insbesondere im Sportbereich, wo das Auftreten von hohen Kräften gewöhnlich ist, sind Vorrichtungen mit großen Abmessungen jedoch unpraktisch. Von der Körperoberfläche hervorstehende Vorrichtungen können den Anwender und Dritte behindern.

US 4,471,538 A zeigt eine stoßdämpfende Vorrichtung mit rheopexer Flüssigkeit.

### Darstellung der Erfindung

Ausgehend von dem bekannten Stand der Technik ist es eine Aufgabe der vorliegenden Erfindung, eine verbesserte Vorrichtung zur Stabilisierung von Körpergelenken, Muskeln und Sehnen anzugeben.

Diese Aufgabe wird mittels einer Vorrichtung mit den Merkmalen des Anspruchs 1 gelöst. Vorteilhafte Ausgestaltungen ergeben sich aus den Unteransprüchen.

Entsprechend wird eine Vorrichtung zur Stabilisierung von Körpergelenken, Muskeln und Sehnen angegeben, welche einen Aufnahmekörper, wobei der Aufnahmekörper mit einem dilatanten Fluid gefüllt ist und der Aufnahmekörper sich in länglicher Form von einem ersten Ende hin zu einem zweiten Ende erstreckt, und genau einen faserförmigen Zugkörper zum Einleiten einer äußeren Kraft in die Vorrichtung, umfasst. Erfindungsgemäß ist ein Kompressionskörper, relativ zum Aufnahmekörper in diesem bewegbar angeordnet und mit dem genau einen faserförmigen Zugkörper verbunden, wobei der Kompressionskörper einen Innenraum des Aufnahmekörpers in eine erste Kammer und eine zweite Kammer unterteilt, wobei zwischen dem Aufnahmekörper und dem Kompressionskörper ein Durchgang bereitgestellt ist, wobei der Durchgang die erste Kammer mit der zweiten Kammer (24) fluidtechnisch verbindet, und wobei der Kompressionskörper mindestens eine Druckfläche umfasst, welche im Wesentlichen eine Seite der ersten Kammer definiert, wobei die Druckfläche auf das in der ersten Kammer befindliche dilatante Fluid drücken kann.

Bevorzugt umfasst der Kompressionskörper eine flache Druckfläche, wobei die flache Druckfläche im Wesentlichen senkrecht zum Zugkörper verläuft.

Dadurch wird eine adaptive Bewegungsbegrenzung ermöglicht, welche auf der über den Zugkörper sowie den Kompressionskörper in das dilatante Fluid eingeleitete Druckkraft verursachten Schwerverdickung des dilatanten Fluides basiert. Drückt der Kompressionskörper in Folge einer ruckartigen Bewegung auf das dilatante Fluid und erreicht die auf das dilatante Fluid wirkende Kraft einen vorbestimmten Schwellwert, erfährt das dilatante Fluid eine Scherverdickung. In diesem scherverdickten Zustand verhält sich das dilatante Fluid inkompressibel, so dass der Kompressionskörper das dilatante Fluid nicht weiterhin verdrängen und sich weiter hin zu dem zweiten Ende des Aufnahmekörpers bewegen kann. Entsprechend führt das inkompressible Verhalten des dilatanten Fluides zu einer Bewegungsbegrenzung der Komponenten der Vorrichtung, welche wiederum zu einer Bewegungsbegrenzung der entsprechenden Körperteile des Anwenders führt.

Da die Bewegungsbegrenzung der Vorrichtung durch das inkompressible Verhalten des dilatanten Fluids bei Druckkräften, welche auf unphysiologischen Bewegungen basieren, hervorgerufen wird, ist eine verhältnismäßig kleine Dimensionierung der Vorrichtung möglich. Um den gewünschten Effekt der Bewegungsbegrenzung zu realisieren, sind insbesondere keine vergleichsweise großen Scherflächen nötig wie bei einer Vorrichtung mit einem rein auf Scherung basierenden Wirkprinzip.

Die erste Kammer wird durch den Bereich zwischen dem Kompressionskörper und dem ersten Ende der Aufnahmevorrichtung und die zweite Kammer zwischen dem Kompressionskörper und dem zweiten Ende der Aufnahmevorrichtung gebildet. Bewegt sich der Kompressionskörper aufgrund einer über den Zugkörper einwirkenden Kraft, welche kleiner als der vorbestimmte Schwellwert ist, relativ zum Aufnahmekörper durch das dilatante Fluid, kann das verdrängte dilatante Fluid von einer Kammer in die andere Kammer strömen. Dabei strömt das dilatante Fluid von der zweiten Kammer in die erste Kammer, wenn sich der Kompressionskörper hin zu dem zweiten Ende der Aufnahmevorrichtung bewegt und von der ersten Kammer in die zweite Kammer, wenn sich der Kompressionskörper hin zu dem ersten Ende der Aufnahmevorrichtung bewegt.

Der Aufnahmekörper sowie der aus dem Aufnahmekörper herausragende Zugkörper können jeweils über eine Halterung an entsprechenden Körperstellen des Anwenders befestigt werden. Mittels des Durchgangs kann das durch den Kompressionskörper verdrängte dilatante Fluid von einer Kammer in die entsprechend andere Kammer strömen. Der Durchgang kann dabei in einer den Kompressionskörper umlaufenden Weise bereitgestellt werden, in welcher der Kompressionskörper eine geringere Querschnittfläche aufweist. Beispielsweise kann der Außendurchmesser des Kompressionskörpers entsprechend kleiner als der Innendurchmesser des Aufnahmekörpers ausgeführt sein. Alternativ kann der Durchgang in Form einer Nut in dem Kompressionskörper bereitgestellt werden.

In einer bevorzugten Ausgestaltung stehen der Kompressionskörper und das dilatante Fluid derart miteinander in Wirkverbindung, dass bei einer auf den Kompressionskörper wirkenden Kraft unterhalb eines vorbestimmten Schwellwerts der Kompressionskörper das dilatante Fluid verdrängen kann, wobei das dilatante Fluid relativ zum Kompressionskörper innerhalb des Aufnahmekörpers strömen kann, und bei einer auf den Kompressionskörper wirkenden Kraft größer gleich des vorbestimmten Schwellwerts der Kompressionskörper auf das dilatanten Fluid drücken kann, wobei sich das von dem Kompressionskörper komprimierte dilatante Fluid Festkörpercharakter aufweist, und wobei ein Strömen des dilatanten Fluids relativ zum Kompressionskörper nicht möglich ist.

Der Schwellwert, welcher die Kraft festlegt, ab welcher die Vorrichtung keine Relativbewegung zwischen Aufnahmekörper und Kompressionskörper mehr zulässt, kann anwendungsspezifisch festgelegt werden. Dabei kann der Schwellwert über die Dimensionierung der Vorrichtung sowie über die Beschaffenheit des dilatanten Fluides eingestellt werden. In der Regel betrifft der Schwellwert den Bereich in einem Kraftwegdiagramm der Vorrichtung, in welchem ein Dilatanzsprung zu verzeichnen ist.

Bei der Vorrichtung zur Stabilisierung von Körpergelenken, Muskeln und Sehnen unterteilt der Kompressionskörper einen Innenraum des Aufnahmekörpers in eine erste Kammer und eine zweite Kammer, wobei das Volumen der ersten Kammer und der zweiten Kammer von der Position des Kompressionskörpers in dem Aufnahmekörper abhängt.

In einer Weiterbildung weist der Kompressionskörper mindestens einen Strömungskanal auf, wobei der Strömungskanal die erste Kammer mit der zweiten Kammer fluidtechnisch verbindet. Durch die Anzahl und Größe der Strömungskanäle ist es möglich, den vorbestimmten Schwellwert festzulegen. Das Bereitstellen von Strömungskanälen im Kompressionskörper ermöglicht eine Verzögerung der Scherverdickung in Bezug auf die Größe der einwirkenden Kraft. Somit ist eine Modifikation der Vorrichtung in Bezug auf den Schwellwert durch die Anzahl und Dimensionierung der Strömungskanäle in dem Kompressionskörper möglich.

In einer weiter bevorzugten Ausführungsform verjüngt sich der Querschnitt des Aufnahmekörpers im Bereich eines Endabschnitts des Aufnahmekörpers hin zu dem zweiten Ende des Aufnahmekörpers, wobei der Kompressionskörper mittels des Zugkörpers in Richtung des zweiten Endes des Aufnahmekörpers ziehbar ist.

Dadurch ist es möglich, dass der Effekt der Scherverdickung in Bezug auf die Größe der einwirkenden Kraft umso früher einsetzt, umso näher sich der Kompressionskörper an dem zweiten Ende befindet. Dadurch kann eine Vorrichtung mit einem variablen Schwellwert bereitgestellt werden, wobei der Schwellwert von der Größe des Querschnitts des Aufnahmekörpers abhängt. Der Schwellwert, ab welchem der Effekt der Scherverdickung einsetzt, ist um so kleiner, umso geringer die Querschnittfläche des Aufnahmekörpers an der entsprechenden Position des Kompressionskörpers ist.

In einer weiter bevorzugten Ausführungsform ist der Kompressionskörper kegelförmig, wobei die Kegelspitze hin zu dem ersten Ende des Aufnahmekörpers gerichtet ist. Dadurch kann die Rückführung des Kompressionskörpers in eine Ausgangsposition begünstigt werden. Zur Rückführung des Kompressionskörpers in die Ausgangsposition kann beispielsweise ein elastisches Rückführelement in der Aufnahmevorrichtung angeordnet sein, welches das erste Ende der Aufnahmevorrichtung mit dem Kompressionskörper verbindet. Wird der Kompressionskörper durch den Zugkörper aufgrund einer einwirkenden Kraft aus der Ausgangsposition ausgelenkt und hin zu dem zweiten Ende bewegt, wird das Rückstellelement gedehnt. Wird die auf den Kompressionskörper wirkende Kraft reduziert oder fällt diese Kraft weg, bewegt das elastische Rückstellelement den Kompressionskörper wieder zurück in seine Ausgangsposition. Die keilförmige Ausgestaltung des Kompressionskörpers begünstigt dabei das Gleiten des Kompressionskörpers durch das dilatante Fluid.

In einer weiter bevorzugten Ausgestaltung weist der Kompressionskörper mindestens ein Projektionselement zum Vergrößern der Druckfläche des Kompressionskörpers auf, wobei das Projektionselement über ein Gelenk mit dem Kompressionskörper verbunden ist. Dadurch kann ein variierender Schwellwert und somit eine variierende Dämpfungswirkung der Vorrichtung beim Eindrücken des Scherkörpers in die Aufnahme ermöglicht werden. Das mindestens eine Projektionselement ist in einer Ausgangsposition am Kompressionskörper anliegend angeordnet. Wird der Kompressionskörper aufgrund einer Krafteinwirkung durch den Zugkörper hin zu dem zweiten Ende des Aufnahmekörpers bewegt, und überschreitet der Kompressionskörper dabei eine vorbestimmte Relativgeschwindigkeit gegenüber dem umströmenden dilatanten Fluid, bewegt sich das mindestens eine Projektionselement aus der Ausgangsposition, so dass die Druckfläche, mit welcher der Kompressionskörper auf das dilatante Fluid drückt, im Vergleich zu der Druckfläche, bei welcher sich das mindestens eine Projektionselement in der Ausgangsposition befindet, vergrößert ist.

Ferner ist das mindestens eine Projektionselement derart an dem Kompressionskörper angeordnet, dass bei einer Rückstellbewegung des Kompressionskörpers in Richtung des ersten Endes des Aufnahmekörpers das mindestens eine Projektionselement in seine Ausgangsposition zurückkehrt. Die Rückstellbewegung kann dabei durch das umströmende dilatante Fluid begünstigt werden.

In einer bevorzugten Ausgestaltung weist der Aufnahmekörper am zweiten Ende einen Dichtkörper zum Abdichten des Innenraums des Aufnahmekörpers gegenüber der Umgebung auf, wobei der Dichtkörper eine Öffnung zur Aufnahme des Zugkörpers umfasst, und der Durchmesser der Öffnung verstellbar ist.

Dadurch kann zusätzlich auf die Eigenschaften der Scherverdickung Einfluss genommen werden, insbesondere die Leichtgängigkeit der Vorrichtung, das heißt, die Leichtgängigkeit, mit welcher sich der Zugkörper relativ zur Öffnung in dem Dichtkörper bewegt, ist dadurch einstellbar. Umso größer der Durchmesser der Öffnung ist, umso leichtgängiger kann sich der Kompressionskörper relativ zum Aufnahmekörper bewegen.

Umgekehrt bedeutet das, dass umso kleiner der Durchmesser der Öffnung ist, umso schwerfälliger sich der Kompressionskörper relativ zum Aufnahmekörper bewegt. Entsprechend ist es möglich, die Intensität, mit welcher die Vorrichtung in eine Bewegung eines zu stabilisierenden Körperteils eingreift, anwendungsspezifisch anzupassen. Beispielsweise kann mit fortschreitendem Heilungsverlauf eines zu stabilisierenden Körperteils der Durchmesser der Öffnung schrittweise vergrößert werden, so dass die Vorrichtung immer umfangreichere Bewegungen des Körperteils zulässt.

Ferner kann die Einstellbarkeit des Durchmessers der Öffnung auch zur Anpassung an das entsprechende, zu stabilisierende Körperteil sowie an Größe und Gewicht des Anwenders angepasst werden.

Die Verstellbarkeit des Durchmessers der Öffnung kann beispielsweise über einen Schieber realisiert werden. Alternativ können Dichtungen mit unterschiedlich großen Öffnungen bereitgestellt werden, wobei je nach Anwendungsfall eine Dichtung mit einer geeigneten Öffnung in den Aufnahmekörper eingesetzt werden kann.

In einer bevorzugten Ausführungsform ist der Zugkörper faserförmig. Dadurch kann die Vorrichtung verhältnismäßig klein dimensioniert werden. Beispielsweise kann der Zugkörper in Form einer synthetischen Faser ausgebildet sein. Ein solcher Zugkörper benötigt vergleichsweise wenig Bauraum und kann vergleichsweise flach auf dem Körper des Anwenders geführt werden. Dies ist insbesondere für Anwendungen im Sportbereich vorteilhaft, bei welchen die Vorrichtung möglichst flach und möglichst klein ausgeführt sein soll, um den Anwender oder dritte Personen nicht zu behindern.

Bekannte synthetische Fasern weisen Festigkeitseigenschaften auf, welche den Anforderungen der Vorrichtung, insbesondere der Stabilisierung von Körpergelenken, Muskeln und Sehnen gerecht werden.

Darüber hinaus ermöglicht ein faserförmiger Zugkörper eine insgesamt vergleichsweise beweglichere Ausgestaltung der Vorrichtung. Insbesondere bei Bewegungen des zu stabilisierenden Körperteils im physiologischen Bereich, in welchem die auf den Kompressionskörper wirkende Kraft unterhalb eines vorbestimmten Schwellwerts liegt, lässt der faserförmige Zugkörper mehr Bewegungsfreiheit zu als ein vergleichbarer steifer Zugkörper.

In einer Weiterbildung ist der Aufnahmekörper flexibel ausgebildet. Der Aufnahmekörper kann dabei ein plastisches oder elastisches Material umfassen, welches eine Anpassung des Aufnahmekörpers an den zu stabilisierende Körperteil ermöglicht. Wird der Aufnahmekörper beispielsweise im Bereich eines Fußknöchels des Anwenders angebracht, kann der Aufnahmekörper aufgrund seiner flexiblen Eigenschaften an die Kontur des Knöchels angepasst werden. Der Aufnahmekörper kann dann mittels entsprechenden Befestigungselementen in einer an das entsprechende Körperteil angepassten Form an dem Körperteil fixiert werden.

Der flexible Aufnahmekörper besitzt den weiteren Vorteil, dass er sich eventuellen Knickbewegungen des zu stabilisierenden Körperteils anpassen kann. Entsprechend ist der Aufnahmekörper in der Lage, eine Biegebewegung des zu stabilisierenden Körperteils nachzuvollziehen. Dadurch kann sichergestellt werden, dass die Vorrichtung in unterschiedlichen Positionen des zu stabilisierenden Körperteils flach auf dem Körperteil aufliegt.

Darüber hinaus kann mittels einer flexiblen Ausgestaltung des Aufnahmekörpers ein Massageeffekt genutzt werden. Bewegt sich der Kompressionskörper in einem physiologischen Bereich, das heißt, bei einer auf den Kompressionskörper wirkenden Kraft unterhalb des vorbestimmten Schwellwerts, relativ zum Aufnahmekörper, kann an der Stelle des Aufnahmekörpers, an welcher sich der Kompressionskörper befindet, ein Druck auf das an die Vorrichtung angrenzende Körperteil des Anwenders aufgebracht werden. Durch die Bewegung des Kompressionskörpers relativ zu dem flexiblen Aufnahmekörper, wandert auch die mittels der Vorrichtung an das angrenzende Körperteil aufgebrachte Druckstelle. Entsprechend kann der angrenzende Körperteil des Anwenders durch die Bewegung des Kompressionskörpers massiert werden. Entsprechend kann die Vorrichtung beispielsweise zur Lymphdrainage verwendet werden.

In einer weiter bevorzugten Ausführungsform weist der Aufnahmekörper eine gebogene Form auf, welche an ein Körpergelenk, einen Muskel oder eine Sehne angepasst ist. Entsprechend kann die Vorrichtung anwendungsspezifisch geformt sein. Beispielsweise kann der Aufnahmekörper um einen Knöchel des Anwenders herum gebogen sein. Somit ist es möglich, die Vorrichtung an die Oberfläche eines zu stabilisierenden Körperteils anzupassen. Insbesondere kann durch eine gebogene Ausgestaltung des Aufnahmekörpers das Abstehen der Vorrichtung von der Körperoberfläche des Anwenders reduziert werden.

In einer weiter bevorzugten Weiterbildung weist der Aufnahmekörper eine Breite beziehungsweise einen Durchmesser von maximal 15 mm auf. Die Vorrichtung kann entsprechend verhältnismäßig klein dimensioniert werden, wodurch sie sich besonders für den Einsatz im Sportbereich eignet.

In einer weiter bevorzugten Ausführungsform sind innerhalb des Aufnahmekörpers Feststoffe bereitgestellt, wobei die Feststoffe mit dem dilatanten Fluid vermischt sind. Durch die Feststoffe können die Eigenschaften des dilatanten Fluides beeinflusst werden. Insbesondere das inkompressible Verhalten des dilatanten Fluides im Fall einer vom Kompressionskörper ausgehenden Druckkraft, welche gleich oder größer des vorbestimmten Schwellwerts ist, kann durch die Zugabe von Feststoffen gesteigert werden. Die Feststoffe können beispielsweise Partikel aus Kunststoff, insbesondere Polymerpartikel sein. Diese Kunststoffpartikel können beispielsweise pulverförmig innerhalb des Aufnahmekörpers bereitgestellt sein.

In einer bevorzugten Weiterbildung ist der Aufnahmekörper eine Hohlfaser. Dadurch ist es möglich, die Vorrichtung in Textilprodukte, wie zum Beispiel Bandagen, Pelotten, Handschuhe, Schuhe und dergleichen zu integrieren. Das Wirkprinzip auf welchem die Vorrichtung beruht, ermöglicht eine besonders kleine Bauweise. Integriert in eine Hohlfaser ist so eine Vorrichtung zur Bewegungsbegrenzung möglich, welche sich insbesondere für den Sportbereich eignet.

Die oben genannte Aufgabe wird auch durch ein System mit den Merkmalen des Anspruchs 13 gelöst. Entsprechend wird ein System angegeben, welches aus einer Vielzahl der oben beschriebenen Vorrichtungen besteht, wobei die Aufnahmekörper im Wesentlichen parallel zueinander angeordnet sind.

Dadurch ist es möglich, trotz der vergleichsweise kleinen Dimensionierung der einzelnen Vorrichtungen, entsprechende Körperteile ausreichend zu stabilisieren. Die auf das System wirkenden Kräfte können kaskadenförmig über die Vielzahl der Vorrichtungen verteilt werden.

Eine Vielzahl verhältnismäßig klein dimensionierter Vorrichtungen ermöglicht eine flache Bauweise, so dass das System unwesentlich weit von dem zu stabilisierenden Körperteil hervorsteht.

In einer bevorzugten Ausführungsform ist die Vielzahl von Vorrichtungen von einer elastischen Hülle umgeben, wobei die elastische Hülle fluiddicht ist. Die elastische Hülle ermöglicht einen Massageeffekt des Körperteils, auf dem das System aufliegt. Des Weiteren kann das System an die Form des zu stabilisierenden Körperteils angepasst werden. Durch die fluiddichte Hülle können geringere Anforderungen an die Abdichtung der einzelnen Vorrichtungen gestellt werden. So stellt die fluiddichte Hülle sicher, dass der Anwender nicht mit dilatantem Fluid in Berührung kommt.

In einer Weiterbildung umfasst die Vielzahl von Vorrichtungen einen zumindest teilweise umlaufenden Flansch. Der Flansch erleichtert die Befestigung des Systems an dem Körper des Anwenders. So kann der Flansch beispielsweise unmittelbar auf dem Körper des Anwenders aufgeklebt werden. Alternativ kann der Flansch an den Körper des Anwenders getapet werden. Des Weiteren kann der Flansch mittels eines Halteelements, wie zum Beispiel einem Gummiband, einer Schelle, und dergleichen an dem Körper des Anwenders befestigt werden.

Die teilweise umlaufende Ausgestaltung des Flanschs ermöglicht eine ausreichende Krafteinleitung von den angrenzenden Körperteilen des Anwenders in das System, bestehend aus einer Vielzahl von Vorrichtungen.

In einer weiter bevorzugten Ausführungsform besteht ein System aus einer Vielzahl der oben beschriebenen Vorrichtungen, wobei die Vorrichtungen in Reihe geschaltet sind. Dadurch kann die vom Anwender ausgehende Kraft auf mehrere Vorrichtungen verteilt werden. Somit ist eine deutlich kleiner Bauweise der einzelnen Vorrichtungen möglich.

### Kurze Beschreibung der Figuren

Bevorzugte weitere Ausführungsformen und Aspekte der vorliegenden Erfindung werden durch die nachfolgende Beschreibung der Figuren näher erläutert. Dabei zeigen:
- Figur 1a: schematisch eine Schnittansicht einer Vorrichtung zur Stabilisierung von Körpergelenken, Muskeln und Sehnen in einem Zustand, in welchem ein dilatantes Fluid relativ zu einem Kompressionskörper fließen kann,
- Figur 1b: schematisch eine Schnittansicht der Vorrichtung aus Figur 1A, in einem Zustand, in welchem sich das dilatante Fluid inkompressibel verhält.
- Figur 2a: schematisch eine Schnittansicht einer Vorrichtung zur Stabilisierung von Körpergelenken, Muskeln und Sehnen mit einem sich verjüngenden Aufnahmekörper in einem Zustand, in welchem sich der Kompressionskörper relativ zum Aufnahmekörper bewegen kann,
- Figur 2b: schematisch eine Schnittansicht der Vorrichtung aus Figur 2A, in einem Zustand, in welchem sich der Kompressionskörper nicht relativ zum Aufnahmekörper bewegen kann,
- Figuren 3a und 3b: schematisch eine Schnittansicht einer Vorrichtung zur Stabilisierung von Körpergelenken, Muskeln und Sehnen, wobei der Kompressionskörper Projektionselemente umfasst,
- Figuren 4a, 4b und 4c: jeweils schematisch eine Schnittansicht einer Vorrichtung zur Stabilisierung von Körpergelenken, Muskeln und Sehnen, wobei die Vorrichtung zwei Zugkörper aufweist,
- Figur 5: schematisch eine Schnittansicht einer Vorrichtung zur Stabilisierung von Körpergelenken, Muskeln und Sehnen, wobei zwei Endabschnitte des Aufnahmekörpers eine Verjüngung aufweisen und der Kompressionskörper über zwei Zugkörper bewegbar ist,
- Figur 6a, 6b und 6c: schematische Detailansichten eines Kompressionskörpers mit Strömungskanälen,
- Figuren 7a und 7b: schematische Detailansichten eines Kompressionskörpers mit Strömungskanälen in der Umfangsfläche,
- Figur 8: schematisch eine Schnittansicht einer Pelotte, welche ein System, bestehend aus einer Vielzahl von Vorrichtungen zur Stabilisierung von Körpergelenken, Muskeln und Sehnen, umfasst,
- Figur 9: schematisch eine Schnittansicht zwei in Reihe geschalteter Vorrichtungen, und
- Figur 10: schematisch eine Hohlfaser, in welcher eine Vielzahl an Vorrichtungen aufgenommen ist.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente mit identischen Bezugszeichen bezeichnet. Um Redundanzen zu vermeiden, wird auf eine wiederholte Beschreibung dieser Elemente in der nachfolgenden Beschreibung teilweise verzichtet.

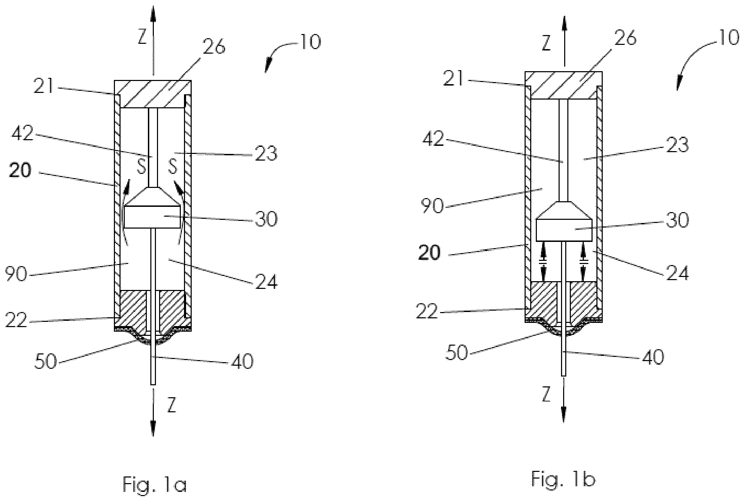

Figuren 1a und 1b ist eine Vorrichtung 10 zum Stabilisierung von Körpergelenken, Muskeln und Sehnen zu entnehmen. Die Vorrichtung 10 umfasst einen Aufnahmekörper 20, welcher sich von einem ersten Ende 21 hin zu einem zweiten Ende 22 erstreckt. Der Aufnahmekörper 20 ist mit einem dilatanten Fluid 90 gefüllt.

In dem Aufnahmekörper 20 ist ein Kompressionskörper 30 angeordnet, welcher den Innenraum des Aufnahmekörpers 20 in eine erste Kammer 23 und eine zweite Kammer 24 teilt. An dem Kompressionskörper 30 ist ein Zugkörper 40 befestigt, welcher längs durch die zweite Kammer 24 verläuft und am zweiten Ende 22 des Aufnahmekörpers 20 aus diesem heraustritt. Das in den Figuren 1a und 1b gezeigte freie Ende des Zugkörpers 40 kann mit einem Körperteil eines Anwenders verbunden werden, um eine von dem Körperteil ausgehende Zugkraft über den Zugkörper 40 auf den Kompressionskörper 30 zu übertragen.

Der Zugkörper 40 ist faserförmig ausgestaltet und kann lediglich Zugkräfte übertragen. Der in den Figuren 1a und 1b gezeigte Zugkörper 40 ist aus einer Kunststofffaser gebildet. Alternativ kann der Zugkörper auch Naturfasern oder andere gängige Faserwerkstoffe umfassen.

Der Aufnahmekörper 20 ist im Bereich des ersten Endes 21 durch einen Verschluss 26 verschlossen, so dass sich in dem Aufnahmekörper 20 befindliches dilatantes Fluid in dem Aufnahmekörper 20 gehalten werden kann. Der Verschluss 26 ist über ein Rückstellelement 42 mit dem Kompressionskörper 30 verbunden. Das Rückstellelement 42 ist elastisch und dazu in der Lage, den Kompressionskörper 30 nach einer durch den Zugkörper 40 erfahrenen Auslenkung wieder in eine Ausgangsposition innerhalb des Aufnahmekörpers 20 zu befördern.

Im Bereich des zweiten Endes 22 des Aufnahmekörpers 20 ist ein Dichtkörper 50 angeordnet, welcher den Innenraum des Aufnahmekörpers 20 gegenüber dem Zugkörper 40 abdichtet. Dabei weist der Dichtkörper 50 eine Öffnung 52 auf, durch welche der Zugkörper 40 aus dem Innenraum des Aufnahmekörpers 20 heraustritt.

Der Kompressionskörper 30 kann mittels des Zugkörpers 40 durch das dilatante Fluid in Richtung des zweiten Endes 22 bewegt werden. Dabei ist der Aufnahmekörper 20 mit einer ersten Körperstelle verbunden und der Zugkörper 40 mit einer zweiten Körperstelle verbunden, wobei sich die erste Körperstelle und die zweite Körperstelle relativ zueinander bewegen können. Die Vorrichtung 10 kann anwendungsspezifisch dimensioniert werden, so dass die Vorrichtung 10 physiologische Bewegungen des Anwenders zulässt. Wird der Kompressionskörper 30 im Rahmen einer physiologischen Bewegung mittels des Zugkörpers 40 in Richtung des zweiten Endes 22 bewegt, kann dilatantes Fluid 90 durch einen umlaufenden Spalt zwischen dem Aufnahmekörper 20 und dem Kompressionskörper 30 von der zweiten Kammer 24 in die erste Kammer 23 strömen, wie in Figur 1a gezeigt. Die Pfeile stellen dabei die Strömungsrichtung S des dilatanten Fluids dar. Wirkt keine Kraft mehr über den Zugkörper 40 auf den Kompressionskörper 30, so kann der Kompressionskörper 30 mittels des Rückstellelements 42 wieder in die Ausgangsposition zurückbewegt werden, wobei dilatantes Fluid durch den umlaufenden Spalt zwischen dem Aufnahmekörper 20 und dem Kompressionskörper 30 von der ersten Kammer 23 in die zweite Kammer 24 strömt.

Dieses Verhalten der Vorrichtung 10 tritt bei auf den Kompressionskörper 30 wirkenden Kräften auf, die einen vorbestimmten Schwellwert nicht überschreiten. Der Schwellwert kann dabei über die Dimensionierung der Vorrichtung 10, die Zusammensetzung des dilatanten Fluides 90, die Größe der Öffnung 52, die Größe des Spalts zwischen dem Aufnahmekörper 20 und dem Kompressionskörper 30 und dergleichen beeinflusst werden.

Wird, wie in Figur 1b gezeigt, eine unphysiologische Kraft, welche gleich oder größer als der vorbestimmte Schwellwert ist, über den Zugkörper 40 in den Kompressionskörper 30 eingeleitet, so führt die von dem Kompressionskörper 30 auf das in der zweiten Kammer 24 befindliche dilatante Fluid 90 aufgebrachte Druckkraft zu einer Scherverdickung des sich in der zweiten Kammer 24 befindlichen dilatanten Fluides 90. Dabei weist das sich in der zweiten Kammer 24 befindliche dilatante Fluid 90 kurzzeitig einen Festkörpercharakter auf, so dass sich das in der zweiten Kammer 24 befindliche dilatante Fluid 90 wie ein Festkörper verhält. Der feste Zustand des sich in der zweiten Kammer 24 befindlichen dilatante Fluids 90 blockiert jegliche Bewegung des Kompressionskörpers 30 in Richtung des zweiten Endes des Aufnahmekörpers 20. Dadurch können sich die beiden, über die Vorrichtung 10 verbundenen Körperteile nicht weiter auseinander bewegen. Darüber hinaus wird auch ein Strömen des dilatanten Fluides von der zweiten Kammer durch den Spalt zwischen dem Aufnahmekörper 20 und dem Kompressionskörper 30 hin zu der ersten Kammer 23 unterbunden.

Der in den Figuren 1a und 1b gezeigte Kompressionskörper 30 weist eine flache Druckoberfläche auf, welche im Wesentlichen senkrecht zu der Zugrichtung Z verläuft. Die Druckfläche definiert einen Teil einer inneren Oberfläche der zweiten Kammer 24. Die Druckkraft, die zu der Verfestigung des dilatanten Fluids 90 in der zweiten Kammer führt, wie in Figur 1b gezeigt, wird von der Druckfläche auf das dilatante Fluid 90 übertragen.

Darüber hinaus weist der in den Figuren 1a und 1b gezeigte Kompressionskörper 30 eine kegelförmige Seite auf, welche dem ersten Ende 21 gegenüberliegt. Die kegelförmige Kontur des Kompressionskörpers 30 erleichtert die Rückstellung des Kompressionskörpers 30 in die Ausgangsposition. Entsprechend begünstigt die kegelförmige Fläche des Kompressionskörpers 30 die Bewegung durch das dilatante Fluid 90 hin zu dem ersten Ende 21.

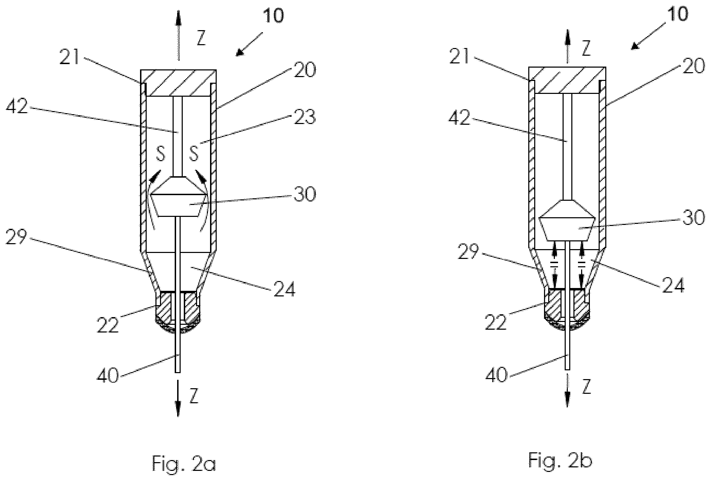

Figuren 2a und 2b unterscheiden sich dadurch von den Figuren 1a und 1b, dass der Aufnahmekörper 20 im Bereich des zweiten Endes 22 einen sich verjüngenden Endabschnitt 29 aufweist. Dabei verläuft die seitliche Oberfläche des Kompressionskörpers 30 derart, dass sie parallel zu dem Endabschnitt 29 des Aufnahmekörpers 20 ausgerichtet ist.

Dadurch kann der Dämpfungseffekt der Vorrichtung 10 kontinuierlich verstärkt werden. Je näher sich der Kompressionskörper 30 am zweiten Ende 22 befindet, wenn er eine unphysiologische Bewegung erfährt, umso geringer ist der vorbestimmte Schwellwert, ab dem es zu einer Scherverdickung des dilatanten Fluides 90 im Bereich der zweiten Kammer 24 kommt.

Der sich verjüngende Endabschnitt 29 beeinflusst auch das Verhalten der Vorrichtung 10 bei physiologischen Bewegungen, welche über den Zugkörper 40 auf den Kompressionskörper 30 wirken. So kann sich der Kompressionskörper 30 in dem Bereich oberhalb des Endabschnitts 29 vergleichsweise leichtgängig durch das dilatante Fluid 90 bewegen. Bewegt sich der Kompressionskörper 30 hingegen im Bereich des sich verjüngenden Endabschnitts 29 in Richtung des zweiten Endes 22, vergrößert sich der Strömungswiderstand, den der Kompressionskörper 30 durch das dilatante Fluid erfährt, mit Abnahme des Abstands zwischen dem Kompressionskörper 30 und dem zweiten Ende 22.

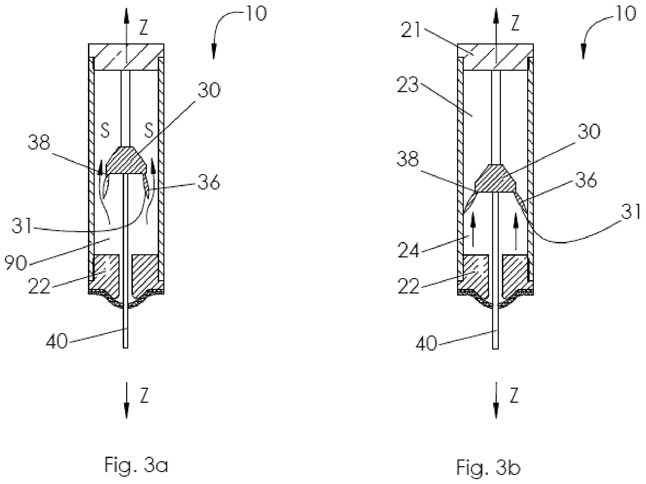

Figuren 3a und 3b ist eine Vorrichtung 10 zu entnehmen, welche sich von der in den Figuren 1a und 1b gezeigten Vorrichtungen dadurch unterscheidet, dass der Kompressionskörper 30 Projektionselemente 36 aufweist. Figur 3a ist ein Kompressionskörper 30 mit anliegenden Projektionselementen 36 zu entnehmen. Die Projektionselemente 36 sind über Gelenke 38 mit dem Kompressionskörper 30 verbunden.

Wirken vergleichsweise geringe Kräfte auf den Zugkörper 40, so kann der Kompressionskörper 30 mit anliegenden Projektionselementen 36 durch das dilatante Fluid 90 hin zu dem zweiten Ende 22 bewegt werden. Dabei ist die von dem Kompressionskörper 30 auf das dilatante Fluid 90 wirkende Druckfläche 31 vergleichsweise klein.

Figur 3b ist ein Kompressionskörper 30 mit ausgeklappten Projektionselementen 36 zu entnehmen. Wirkt eine unphysiologische Kraft über den Zugkörper 40 auf den Kompressionskörper 30, werden die Projektionselemente 36 durch das den Kompressionskörper 30 umströmende dilatante Fluid 90 ausgeklappt. Im Vergleich zu der in Figur 3a gezeigten anliegenden Position der Projektionselemente 36 weist der in Figur 3b gezeigte Kompressionskörper 30 eine deutlich größere Druckfläche 31 auf, mit welcher er auf das dilatante Fluid 90 im Bereich der zweiten Kammer 24 drückt. Überschreitet die auf den Kompressionskörper 30 wirkende Kraft einen vorbestimmten Schwellwert, kommt es zu einer Scherverdickung des dilatanten Fluides 90 im Bereich der zweiten Kammer 24.

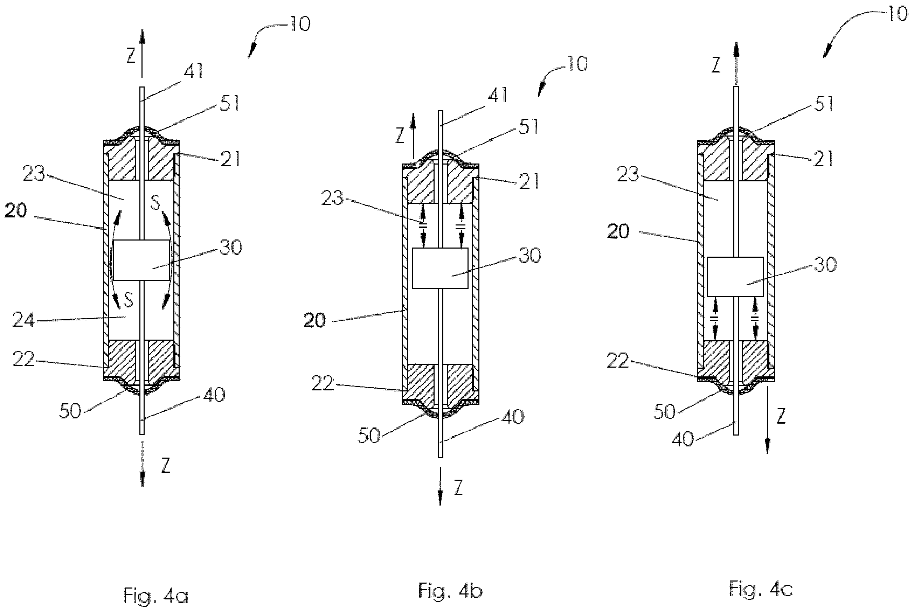

Den Figuren 4a, 4b und 4c ist eine Vorrichtung 10 zu entnehmen, welche sowohl bei einer Bewegung des Kompressionskörpers 30 hin zu dem ersten Ende 21 als auch bei einer Bewegung des Kompressionskörpers 30 hin zu dem zweiten Ende 22 eine Scherverdickung des dilatanten Fluides 90 in der entsprechenden Kammer 23, 24 hervorrufen kann. Der in dem Aufnahmekörper 20 angeordnete Kompressionskörper 30 kann von einem Zugkörper 40 hin zu dem zweiten Ende 22 gezogen werden und von einem Zugkörper 41 hin zu dem ersten Ende 21 gezogen werden. Wirkt eine Kraft, welche aus einer unphysiologischen Bewegung resultiert, über den Zugkörper 41 auf den Kompressionskörper 30 und bewegt diesen hin zu dem Ende 21, so kommt es zu einer Scherverdickung des dilatanten Fluides 90 in der ersten Kammer 23, wie in Figur 4b gezeigt. Wirkt eine Kraft, welche aus einer unphysiologischen Bewegung resultiert, auf den Kompressionskörper 30 und bewegt diesen hin zu dem zweiten Ende 22, so erfährt das dilatante Fluid 90 in der zweiten Kammer 24 eine Scherverdickung, wie in Figur 4c gezeigt. Dabei sind der Zugkörper 41, der Aufnahmekörper 20 und der Zugkörper 40 jeweils an unterschiedlichen Stellen des Körpers des Anwenders befestigt.

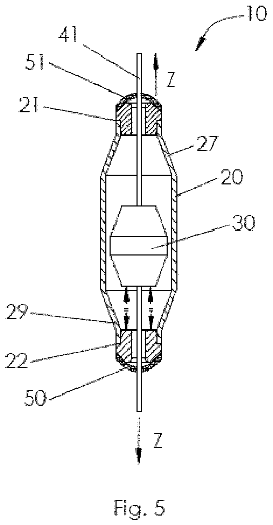

Figur 5 zeigt eine Vorrichtung 10, welche sich von der in den Figuren 4a, 4b und 4c gezeigten Vorrichtung darin unterscheidet, dass der Aufnahmekörper 20 im Bereich des ersten Endes 21 einen sich verjüngenden Endabschnitt 27 und im Bereich des zweiten Endes 22 einen sich verjüngenden Endabschnitt 29 aufweist.

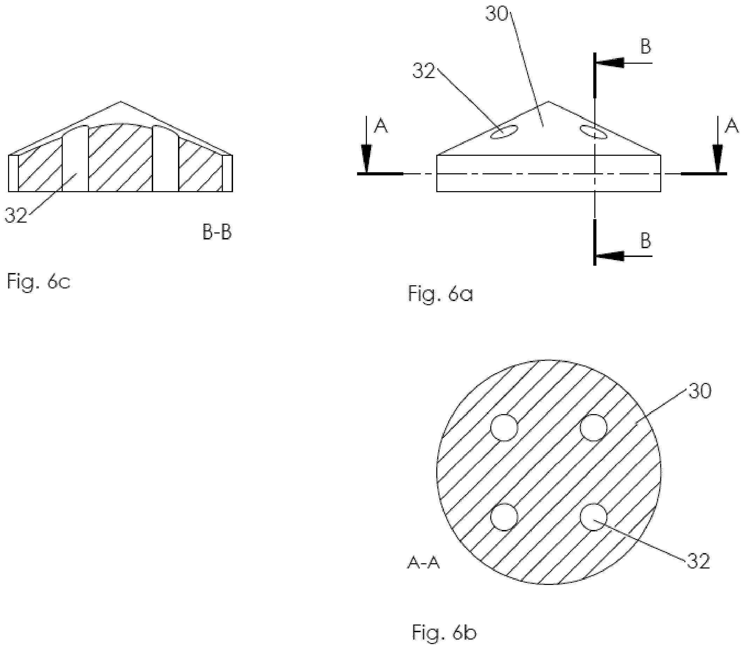

Figur 6a ist eine Seitenansicht eines Kegelkörpers 30 zu entnehmen. Der Kegelkörper 30 weist Strömungskanäle 32 auf, durch welche dilatantes Fluid zwischen der ersten und der zweiten Kammer strömen kann. Figur 6b ist eine Schnittansicht der in Figur 6a gezeigten Schnittlinie A-A zu entnehmen. Der Kompressionskörper 30 weist vier Strömungskanäle 32 auf. Alternativ kann der Kompressionskörper auch einen, zwei, drei, fünf, sechs, sieben und mehr Strömungskanäle aufweisen. Figur 6c ist eine Schnittansicht der in Figur 6a gezeigten Schnittlinie B-B zu entnehmen.

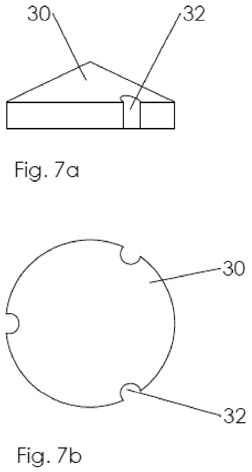

Figur 7a zeigt einen Kompressionskörper 30 mit Strömungskanälen 32, welche in der Form von Nuten in der Umfangsoberfläche des Kompressionskörpers 30 gebildet sind. Figur 7b zeigt eine Draufsicht des Kompressionskörpers 30. Der Kompressionskörper 30 weist drei Strömungskanäle 32 auf.

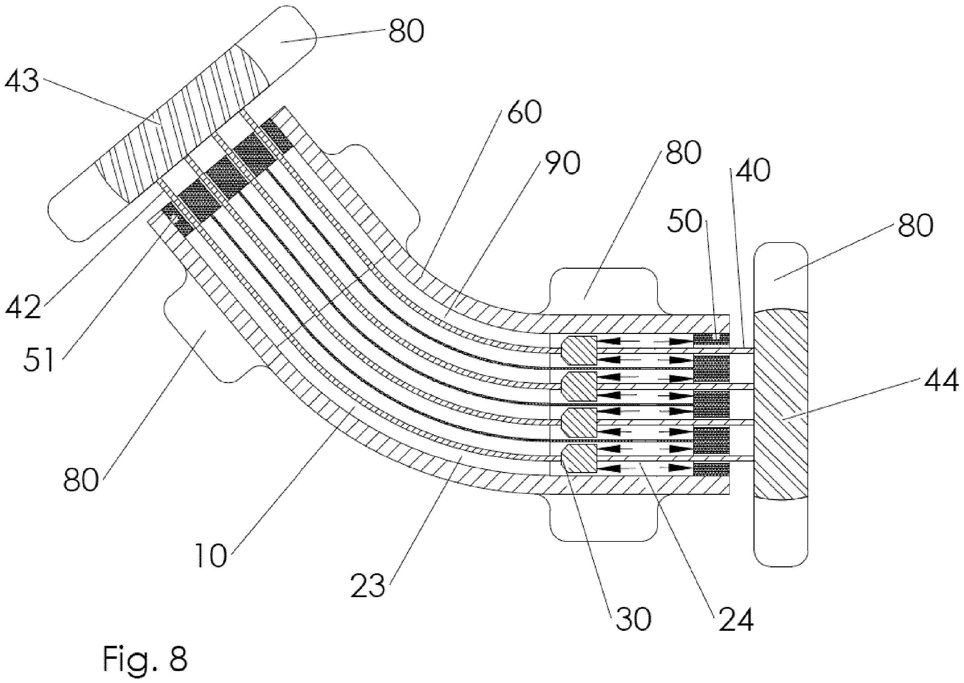

Figur 8 zeigt ein System, bestehend aus Vorrichtungen 10 zur Stabilisierung von Körpergelenken, Muskeln und Sehnen. Dabei sind vier Vorrichtungen 10 nebeneinander angeordnet. Die Aufnahmekörper 20 der einzelnen Vorrichtungen 10 grenzen unmittelbar aneinander an und sind gebogen ausgebildet. In den Vorrichtungen 10 sind jeweils Kompressionskörper 30 angeordnet. Die Kompressionskörper 30 teilen den Innenraum der Aufnahmekörper 20 jeweils in eine erste Kammer 23 und eine zweite Kammer 24. Die Kompressionskörper 30 sind über Zugkörper 40 mit einer Zugkörperbasis 44 verbunden. Darüber hinaus sind die Kompressionskörper 30 über Rückstellelemente 42 mit einer Rückstellbasis 43 verbunden. Die Zugkörper 40 werden durch Dichtkörper 50 gegenüber den zweiten Kammern 24 abgedichtet. Des Weiteren werden die Rückstellelemente 42 über Dichtkörper 51 gegenüber den ersten Kammern 23 abgedichtet.

Die Aufnahmekörper 20 sind von einer elastischen Hülle 60 umgeben. Die elastische Hülle 60 gibt dem System aus Vorrichtungen 10 eine entsprechende Form und stellt darüber hinaus sicher, dass der Anwender nicht mit dem dilatanten Fluid 90 in Berührung kommt. Darüber hinaus kann die elastische Hülle 60 einen Massageeffekt bewirken, wobei der Anwender die Bewegung der Kompressionskörper 30 durch die elastische Hülle 60 wahrnehmen kann.

Ferner umfasst die elastische Hülle 60 abschnittsweise Flansche 80. Die Flansche 80 dienen dabei dazu, das System aus Vorrichtungen 10 auf dem Körper des Anwenders zu befestigen. Beispielsweise können die Flansche 80 auf der dem Körper des Anwenders zugewandten Seite einen Klebstoff aufweisen, mittels welchem das System aus Vorrichtungen 10 unmittelbar auf den Körper des Anwenders geklebt werden kann. Alternativ können die Flansche 80 dazu verwendet werden, um mit Tapes auf dem Körper des Anwenders festgeklebt zu werden.

Dadurch, dass die Flansche 80 jeweils an unterschiedlichen Körperteilen des Anwenders befestigt werden, werden die Relativbewegungen der Kompressionskörper 30 gegenüber den Aufnahmekörpern 20 ermöglicht. Die einzelnen Vorrichtungen 10 verhalten sich im Anwendungsfall wie die in Figur 1a und 1b beschriebene Vorrichtung.

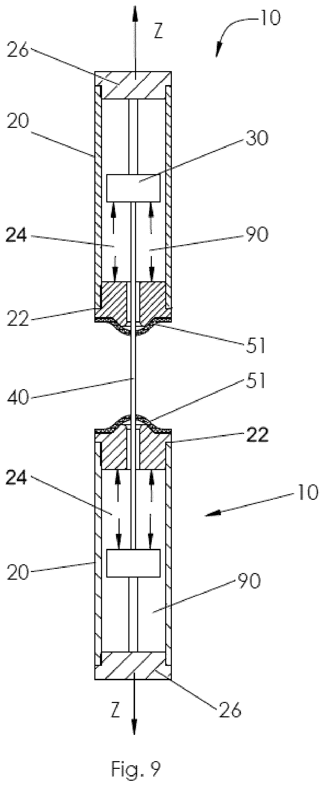

Figur 9 sind zwei Vorrichtungen 10zu entnehmen, welche in Reihe geschaltet sind, wobei die Vorrichtungen 10 weitestgehend der in den Figuren 1a und 1b gezeigten Vorrichtung gleichen. Die beiden Vorrichtungen 10 sind über einen gemeinsamen Zugkörper 40 verbunden. Wirkt auf die beiden Aufnahmekörper 20 eine physiologische Kraft in Zugrichtung Z, bewegen sich die Aufnahmekörper 20 auseinander, wodurch sich der Abstand zwischen den Kompressionskörpern 30 und den zweiten Enden 22 und damit das Volumen der zweiten Kammern 24 verringert.

Wirkt auf die beiden Aufnahmekörper 20 eine unphysiologische Kraft in Zugrichtung Z, kommt es in den zweiten Kammern 24 zu einer Scherverdickung des dilatanten Fluids 90, wodurch sich das Volumen der zweiten Kammern 24 nicht weiter verkleinern kann. Die beiden Aufnahmekörper 20 können sich in diesem Zustand trotz in Zugrichtung Z wirkender Kräfte nicht weiter voneinander entfernen.

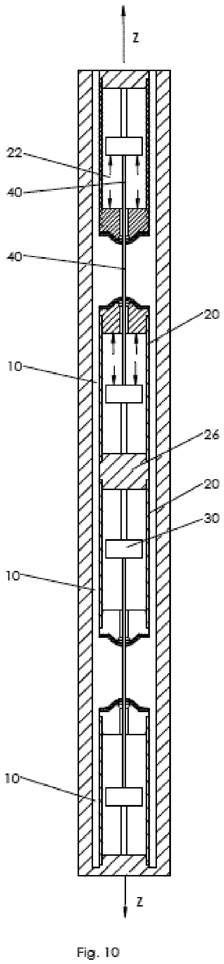

Figur 10 ist eine Hohlfaser 70 zu entnehmen, in deren Inneren eine Vielzahl der Vorrichtungen aus Figuren 1a und 1b angeordnet sind. Die Vorrichtungen 10 sind dabei im Inneren der Hohlfaser in Reihe geschaltet. Dabei teilt sich eine Vorrichtung 10 mit einer benachbarten Vorrichtung 10 einen Zugkörper 40 und mit einer weiteren benachbarten Vorrichtung 10 einen Verschluss 26. Dadurch ist es möglich, die Vorrichtung 10 in Textilprodukte, wie zum Beispiel Bandagen, Pelotten, Handschuhe, Schuhe und dergleichen zu integrieren.

Soweit anwendbar, können alle einzelnen Merkmale, die in den einzelnen Ausführungsbeispielen dargestellt sind, miteinander kombiniert und/oder ausgetauscht werden, ohne den Bereich der Erfindung zu verlassen.

### Bezugszeichenliste

- 10: Vorrichtung

- 20: Aufnahmekörper
- 21: Erstes Ende
- 22: Zweites Ende
- 23: Erste Kammer
- 24: Zweite Kammer
- 26: Verschluss
- 27: Endabschnitt
- 28: Dichtkörper
- 29: Endabschnitt

- 30: Kompressionskörper
- 31: Druckfläche
- 32: Strömungskanal
- 34: Keilspitze
- 36: Projektionselement
- 38: Gelenk

- 40: Zugkörper
- 41: Zugkörper
- 42: Rückstellelement
- 43: Rückstellbasis
- 44: Zugkörperbasis

- 50: Dichtkörper
- 51: Dichtkörper
- 52: Öffnung

- 60: Elastische Hülle

- 70: Hohlfaser
- 80: Flansch

- 90: Dilatantes Fluid

- S: Strömungsrichtung
- Z: Zugrichtung

## Patentansprüche

1. Vorrichtung (10) zur Stabilisierung von Körpergelenken, Muskeln und Sehnen umfassend einen Aufnahmekörper, wobei der Aufnahmekörper (20) mit einem dilatanten Fluid (90) gefüllt ist und der Aufnahmekörper (20) sich in länglicher Form von einem ersten Ende (21) hin zu einem zweiten Ende (22) erstreckt, und einen Kompressionskörper (30), der relativ zum Aufnahmekörper (20) in diesem bewegbar angeordnet ist, wobei der Kompressionskörper (30) einen Innenraum des Aufnahmekörpers (20) in eine erste Kammer (23) und eine zweite Kammer (24) unterteilt, wobei zwischen dem Aufnahmekörper (20) und dem Kompressionskörper (30) ein Durchgang bereitgestellt ist, wobei der Durchgang die erste Kammer (23) mit der zweiten Kammer (24) fluidtechnisch verbindet, und wobei der Kompressionskörper (30) mindestens eine Druckfläche umfasst, welche im Wesentlichen eine Seite der ersten Kammer (24) definiert, wobei die Druckfläche auf das in der ersten Kammer (24) befindliche dilatante Fluid (90) drücken kann, **gekennzeichnet durch** genau einen faserförmigen Zugkörper (40) zum Einleiten einer äußeren Kraft in die Vorrichtung, der mit dem Kompressionskörper (30)verbunden ist.

2. Vorrichtung (10) nach Anspruch 1, **dadurch gekennzeichnet, dass** der Kompressionskörper (30) und das dilatante Fluid (90) derart miteinander in Wirkverbindung stehen, dass bei einer auf den Kompressionskörper (30) wirkenden Kraft unterhalb eines vorbestimmten Schwellwerts der Kompressionskörper (30) das dilatante Fluid (90) verdrängen kann, wobei das dilatante Fluid (90) relativ zum Kompressionskörper (30) innerhalb des Aufnahmekörpers (20) strömen kann, und bei einer auf den Kompressionskörper (30) wirkenden Kraft größer gleich des vorbestimmten Schwellwerts der Kompressionskörper (30) auf das dilatanten Fluid (90) drücken kann, wobei sich das von dem Kompressionskörper komprimierte dilatante Fluid (90) Festkörpercharakter aufweist, und wobei ein Strömen des dilatanten Fluids relativ zum Kompressionskörper (30) nicht möglich ist.

3. Vorrichtung (10) nach Anspruch 1 oder 2, **dadurch gekennzeichnet, dass** der Kompressionskörper (30) mindestens einen Strömungskanal (32) aufweist, wobei der Strömungskanal (32) die erste Kammer (23) mit der zweiten Kammer (24) fluidtechnisch verbindet.

4. Vorrichtung (10) nach einem der vorhergehenden Ansprüche, **dadurch gekennzeichnet, dass** sich der Querschnitt des Aufnahmekörpers (20) im Bereich eines Endabschnitts (27, 29) des Aufnahmekörpers (20) hin zu dem zweiten Ende des Aufnahmekörpers (20) verjüngt, wobei der Kompressionskörper (30) mittels des Zugkörpers (40) in Richtung des zweiten Endes (22) des Aufnahmekörpers (20) ziehbar ist.

5. Vorrichtung (10) nach einem der vorhergehenden Ansprüche, **dadurch gekennzeichnet, dass** der Kompressionskörper (30) kegelförmig ist, wobei die Kegelspitze (34) hin zu dem ersten Ende (21) des Aufnahmekörpers (20) gerichtet ist.

6. Vorrichtung (10) nach einem der vorhergehenden Ansprüche, **dadurch gekennzeichnet, dass** der Kompressionskörper (30) mindestens ein Projektionselement (36) zum Vergrößern der Druckfläche (31) des Kompressionskörpers (30) aufweist, wobei das Projektionselement (36) über ein Gelenk (38) mit dem Kompressionskörper (30) verbunden ist.

7. Vorrichtung (10) nach einem der vorhergehenden Ansprüche, **dadurch gekennzeichnet, dass** der Aufnahmekörper (20) am zweiten Ende (22) einen Dichtkörper (50) zum Abdichten des Innenraums des Aufnahmekörpers (20) gegenüber der Umgebung aufweist, wobei der Dichtkörper (50) eine Öffnung (52) zur Aufnahme des Zugkörpers (40) umfasst, und der Durchmesser der Öffnung (52) verstellbar ist.

8. Vorrichtung (10) nach einem der vorhergehenden Ansprüche, **dadurch gekennzeichnet, dass** der Aufnahmekörper (20) flexibel ausgebildet ist.

9. Vorrichtung (10) nach einem der vorhergehenden Ansprüche, **dadurch gekennzeichnet, dass** der Aufnahmekörper (20) eine gebogene Form aufweist, welche an ein Körpergelenk, einen Muskel oder eine Sehne angepasst ist.

10. Vorrichtung (10) gemäß einem der vorhergehenden Ansprüche, **dadurch gekennzeichnet, dass** innerhalb des Aufnahmekörpers (20) Feststoffe bereitgestellt sind, wobei die Feststoffe mit dem dilatanten Fluid (90) vermischt sind.

11. Vorrichtung (10) gemäß einem der vorhergehenden Ansprüche, **dadurch gekennzeichnet, dass** der Aufnahmekörper (20) eine Hohlfaser (70) ist.

12. System bestehend aus einer Vielzahl von Vorrichtungen (10) nach einem der vorhergehenden Ansprüche, **dadurch gekennzeichnet, dass** die Aufnahmekörper (20) im Wesentlichen parallel zueinander angeordnet sind.

13. System gemäß Anspruch 12, **dadurch gekennzeichnet, dass** die Vielzahl von Vorrichtungen (10) von einer elastischen Hülle (60) umgeben ist, wobei die elastische Hülle (60) fluiddicht ist.

14. System gemäß Anspruch 12 oder 13, **dadurch gekennzeichnet, dass** die Vielzahl von Vorrichtungen (10) einen zumindest teilweise umlaufenden Flansch (80) umfasst.

15. System bestehend aus einer Vielzahl von Vorrichtungen (10) nach einem der Ansprüche 1 bis 11, **dadurch gekennzeichnet, dass** die Vorrichtungen (10) in Reihe geschaltet sind.

## Claims

1. A device (10) for stabilizing body joints, muscles and tendons comprising: a receptacle body (20), wherein the receptacle body (20) is filled with a dilatant fluid (90) and the receptacle body (20) extends in an elongated shape from a first end (21) towards a second end (22), and
a compression body (30), wherein the compression body (30) is arranged in the receptacle body (20) in a relatively movable manner, wherein the compression body (30) divides an interior space of the receptacle body (20) into a first chamber (23) and a second chamber (24), wherein between the receptacle body (20) and the compression body (30) a passage is provided, wherein the passage connects the first chamber (23) with the second chamber (24) fluid-technically, and wherein the compression body (30) comprises at least one compression surface which substantially defines one side of the first chamber (24), wherein the compression surface can press onto the dilatant fluid (90) situated in the first chamber (24), **characterized by** means of exactly one fibrous tension body (40) for initiating an external force into the device, which is connected to the compression body (30).

2. The device (10) according to claim 1, **characterized in that** the compression body (30) and the dilatant fluid (90) are operatively connected together so that in the case of a force acting on the compression body (30) below a predetermined threshold the compression body (30) can displace the dilatant fluid (90), wherein the dilatant fluid (90) may flow relative to the compression body (30) within the receptacle body (20), and in case of a force greater than or equal to the predetermined threshold acting on the compression body (30) the compression body (30) can press onto the dilatant fluid (90), wherein the dilatant fluid (90) compressed by the compression body (30) has the characteristics of a solid body, and wherein a flow of the dilatant fluid (90) relative to the compression body (30) is suppressed.

3. The device (10) according to claim 1 or 2, **characterized in that** the compression body (30) has at least one flow channel (32), wherein the flow channel (32) connects the first chamber (23) with the second chamber (24) fluid-technically.

4. The device (10) according to one of the preceding claims, **characterized in that** the cross section of the receptacle body (20) in the area of an end portion (29) of the receptacle body (20) tapers towards the second end (22) of the receptacle body (20), wherein the compression body (30) can be pulled in the direction of the second end (22) of the receptacle body (20) by means of the tension body (40).

5. The device (10) according to one of the preceding claims, **characterized in that** the compression body (30) is tapered, wherein the taper apex is directed towards the first end (21) of the receptacle body (20).

6. The device (10) according to one of the preceding claims, **characterized in that** the compression body (30) has at least one projection member (36) for increasing the compression surface (31) of the compression body (30), wherein the projection member (36) is connected to the compression body (30) via a joint.

7. The device (10) according to one of the preceding claims, **characterized in that** at the second end (22) the receptacle body (20) has a sealing body (50) for sealing the interior of the receptacle body (20) against the environment, wherein the sealing body (50) comprises an opening (52) for receiving the tension body (40), and wherein the diameter of the opening (52) is adjustable.

8. The device (10) according to one of the preceding claims, **characterized in that** the receptacle body (20) is of a flexible form.

9. The device (10) according to one of the preceding claims, **characterized in that** the receptacle body (20) has a curved shape which is adapted to a body joint, muscle, or tendon.

10. The device (10) according to one of the preceding claims, **characterized in that** solids are provided within the receptacle body (20), wherein the solids are mixed with the dilatant fluid (90).

11. The device (10) according to one of the preceding claims, **characterized in that** the receptacle body (20) is a hollow fiber (70).

12. A system comprising a plurality of the devices (10) according to one of the preceding claims, **characterized in that** the receptacle bodies (20) are arranged substantially parallel to each other.

13. The system according to claim 14, **characterized in that** the plurality of devices (10) is surrounded by means of an elastic sheath (60), wherein the elastic sheath (60) is fluid-tight.

14. The system according to claim 12 or 13, **characterized in that** the plurality of devices (10) comprises a flange (80) which is at least partially circumferential.

15. A system comprising a plurality of the devices (10) according to one of the claims 1 to 11, **characterized in that** the devices (10) are being connected in series.

## Revendications

1. Dispositif (10) de stabilisation d'articulations corporelles, de muscles et de veines comprenant un corps de réception, dans lequel le corps de réception (20) est rempli avec un fluide dilatant (90) et le corps de réception (20) s'étend sous la forme oblongue d'une première extrémité (21) à une seconde extrémité (22),
et un corps de compression (30) qui est agencé de manière mobile dans celui-ci par rapport au corps de réception (20), dans lequel le corps de compression (30) divise un espace intérieur du corps de réception (20) en une première chambre (23) et une seconde chambre (24), dans lequel un passage est fourni entre le corps de réception (20) et le corps de compression (30), dans lequel le passage relie la première chambre (23) à la seconde chambre (24) de manière fluide, et dans lequel le corps de compression (30) comprend au moins une surface de pression qui définit sensiblement un côté de la première chambre (24), dans lequel la surface de pression peut presser sur le fluide (90) dilatant se trouvant dans la première chambre (24), **caractérisé par** précisément un corps de traction (40) fibreux pour l'introduction d'une force extérieure dans le dispositif qui est relié au corps de compression (30).

2. Dispositif (10) selon la revendication 1, **caractérisé en ce que** le corps de compression (30) et le fluide dilatant (90) sont en liaison active entre eux de telle manière qu'en cas de force agissant sur le corps de compression (30) en dessous d'une valeur seuil prédéterminée le corps de compression (30) peut chasser le fluide dilatant (90), dans lequel le fluide dilatant (90) peut s'écouler par rapport au corps de compression (30) à l'intérieur du corps de réception (20), et en cas de force agissant sur le corps de compression (30) supérieure ou égale à la valeur seuil prédéterminée le corps de compression (30) peut presser sur le fluide dilatant (90), dans lequel le fluide dilatant (90) comprimé par le corps de compression présente des caractères de corps solide, et dans lequel un écoulement du fluide dilatant par rapport au corps de compression (30) n'est pas possible.

3. Dispositif (10) selon la revendication 1 ou 2, **caractérisé en ce que** le corps de compression (30) présente au moins un canal d'écoulement (32), dans lequel le canal d'écoulement (32) relie de manière fluide la première chambre (23) à la seconde chambre (24).

4. Dispositif (10) selon l'une quelconque des revendications précédentes, **caractérisé en ce que** la section transversale du corps de réception (20) se rétrécit dans la zone d'une section d'extrémité (27, 29) du corps de réception (20) vers la seconde extrémité du corps de réception (20), dans lequel le corps de compression (30) peut être tiré au moyen du corps de traction (40) en direction de la seconde extrémité (22) du corps de réception (20).

5. Dispositif (10) selon l'une quelconque des revendications précédentes, **caractérisé en ce que** le corps de compression (30) est conique, dans lequel la pointe conique (34) est dirigée vers la première extrémité (21) du corps de réception (20).

6. Dispositif (10) selon l'une quelconque des revendications précédentes, **caractérisé en ce que** le corps de compression (30) présente au moins un élément de projection (36) pour l'agrandissement de la surface de pression (31) du corps de compression (30), dans lequel l'élément de projection (36) est relié par le biais d'une articulation (38) au corps de compression (30).

7. Dispositif (10) selon l'une quelconque des revendications précédentes, **caractérisé en ce que** le corps de réception (20) présente à la seconde extrémité (22) un corps étanche (50) pour rendre étanche l'espace intérieur du corps de réception (20) par rapport à l'environnement, dans lequel le corps étanche (50) comprend une ouverture (52) pour la réception du corps de traction (40), et le diamètre de l'ouverture (52) est réglable.

8. Dispositif (10) selon l'une quelconque des revendications précédentes, **caractérisé en ce que** le corps de réception (20) est réalisé de manière flexible.

9. Dispositif (10) selon l'une quelconque des revendications précédentes, **caractérisé en ce que** le corps de réception (20) présente une forme pliée qui est adaptée à une articulation corporelle, un muscle ou une veine.

10. Dispositif (10) selon l'une quelconque des revendications précédentes, **caractérisé en ce que** des substances solides sont fournies à l'intérieur du corps de réception (20), dans lequel les substances solides sont mélangées au fluide dilatant (90).

11. Dispositif (10) selon l'une quelconque des revendications précédentes, **caractérisé en ce que** le corps de réception (20) est une fibre creuse (70).

12. Système se composant d'une pluralité de dispositifs (10) selon l'une quelconque des revendications précédentes, **caractérisé en ce que** les corps de réception (20) sont agencés sensiblement parallèlement l'un à l'autre.

13. Système selon la revendication 12, **caractérisé en ce que** la pluralité de dispositifs (10) est entourée par une gaine élastique (60), dans lequel la gaine élastique (60) est étanche au fluide.

14. Système selon la revendication 12 ou 13, **caractérisé en ce que** la pluralité de dispositifs (10) comprend une bride (80) au moins partiellement périphérique.

15. Système se composant d'une pluralité de dispositifs (10) selon l'une quelconque des revendications 1 à 11, **caractérisé en ce que** les dispositifs (10) sont montés en série.
